# EUROPEAN PATENT APPLICATION

(11) **EP 4 194 555 A1**
(43) Date of publication of application: **14.06.2023**
(21) Application number: 21213412.6
(22) Date of filing: 09.12.2021
(51) Int. Cl.: C12N 15/115

(54) **DNA APTAMERS INHIBITING BINDING OF UROKINASE TYPE PLASMINOGEN ACTIVATOR (UPA) WITH UROKINASE TYPE PLASMINOGEN ACTIVATOR RECEPTOR (UPAR) AND INHIBITING UPA ACTIVITY**

(71) Applicant: Fraunhofer-Gesellschaft zur Förderung der angewandten Forschung e.V., 80686 München (DE)
(72) Inventor: Menger, Marcus, 14469 Patsdam (DE); Dreymann, Nico, 14469 Potsdam (DE); Wünsche, Julia, 12527 Berlin (DE)
(74) Representative: Altmann Stößel Dick Patentanwälte PartG mbB

(57) **Abstract**

In a first aspect, the invention relates to a DNA aptamer comprising a polynucleotide sequence having at least 70 % identity to a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO: 10 and SEQ ID NO: 11 for use as a medicament, especially for use in treatment of cancer and/or prevention of cancer in a subject. The DNA aptamers are able to inhibit binding of Urokinase type plasminogen activator (uPA) with Urokinase type plasminogen activator receptor (uPAR) and are also able to inhibit activity of Urokinase type plasminogen activator (uPA). A second aspect of the invention relates to the DNA aptamer, used according to the first aspect, itself. A third aspect of the invention is related to a method for identifying a subject benefiting from cancer treatment with a DNA aptamer according to the first aspect and of the second aspect respectively. In a fourth aspect, the invention relates to a kit comprising a DNA aptamer according to the first aspect and of the second aspect respectively.

## Description

In a first aspect, the invention relates to a DNA aptamer comprising a polynucleotide sequence having at least 70 % identity to a sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11 for use as a medicament, especially for use in treatment of cancer and/or prevention of cancer in a subject. A second aspect of the invention relates to the DNA aptamer, used according to the first aspect, itself. A third aspect of the invention is related to a method for identifying a subject benefiting from cancer treatment with a DNA aptamer according to the first aspect and of the second aspect respectively. In a fourth aspect, the invention relates to a kit comprising a DNA aptamer according to the first aspect and of the second aspect respectively.

Urokinase is a serine protease that performs an important role in the fibrinolytic system. It converts the inactive form plasminogen into the catalytically active form plasmin on cell surfaces leading to extracellular proteolysis. It is produced and secreted as a single polypeptide glycosylated zymogen pro-uPA form consisting of 411 amino acids with a N-terminal A-Chain containing a growth factor domain (GFD, amino acids 1-49), a kringle domain (KD, amino acids 50-131) and a C-terminal B-Chain containing the catalytically active serine protease domain (aa 159-411). Between the N-terminal A-Chain and the C-terminal B-Chain is a linker domain (aa 132-158). Due to different proteases pro-uPA undergoes cleavage of the peptide bond between K158 and 1159 to produce the 54kDa catalytic active two chain uPA form (HMW-uPA), which comprises an A chain (SEQ ID No. 18) and a B chain (SEQ ID No. 19), linked by a disulfide bond between cysteine C148 in the A Chain and cysteine C279 in the B chain, the numbering here and in the following being based on the underlying sequence of pro-UPA. Based on different proteases, HMW-uPA can be cleaved a second time in the linker sequence between K135 and K136 into a catalytically active low molecular weight uPA of 33 kDa (LMW-uPA) with the serine protease domain and an inactive amino-terminal fragment that contains the GFD and the KD. LMW-uPA comprises a (residue) A chain (SEQ ID No. 20) and a B chain (SEQ ID No. 21). (Residue) A chain and B Chain in LMW-uPA are connected by a disulfide bond between cysteine C148 in the (residue) A Chain and cysteine C279 in the B Chain - the B chain of HMW-uPA identical with the B chain of LMW-uPA, wherein, also here, the numbering is based on the numbering of the underlying pro-uPA (residue) A Chain and B Chain in LMW-uPA being connected by a disulfide bond between cysteine C148 in the (residue) A Chain and cysteine C279 in the B Chain; the B Chain of HMW-uPA identical with the B Chain of LMW-uPA, based on the numbering of pro-uPA.

Binding of pro-uPA and HMW-uPA through the GFD to the glycolipid-anchored uPA receptor (uPAR) activates the conversion of plasminogen to plasmin on cell surfaces (Mahmood et al., 2018). While pro-uPA has a more limited enzymatic activity it is however able to induce plasmin activation by conformational change in pro-uPA through the binding to uPAR (Higazi et al., 1995), the two-chain HMW-uPA binding to uPAR is 250-fold more potent and therefore the main activator of plasmin (Petersen et al., 1988), leading to extracellular proteolysis. This mediates several physiological and pathological pathways by activating growth factors and promatrix metalloproteases, degrading extracellular matrix components and basement membrane and allowing the formation of new matrix. This promotes all pathophysiological mechanisms in tumorigenesis like tumor angiogenesis, malignant cell proliferation, invasion of surrounding tissues, cell extravasation as well as tumor progression and metastasis (Mekkawy et al., 2014).

Inhibition of the catalytic activity of uPA is caused by the serine protease inhibitors plasminogen activator inhibitors (PAI-1 and PAI-2). Here, PAI-1 is the major inhibitor which makes the conversion to plasmin a highly controlled event. PAI-1 and uPA form a covalently linked complex which is absorbed together with uPAR by endocytosis which leads to degradation of the uPA-PAI-1 complex and releases free uPAR to the extracellular region (Dupont et al., 2009). Independent of proteolytic activity, the uPA-uPAR complex play also a key role in signal transduction by interacting with several adhesion molecules like integrins and vitronectin, cellular receptors and proteins of the extracellular matrix to control cell proliferation, apoptosis, chemotaxis, adhesion and migration (Mekkawy et al., 2014). Many studies have been conducted devising strategies to target uPA in anticancer therapies by inhibition of the proteolytic activity of uPA or by inhibiting the uPA-uPAR interaction. Blocking of the catalytic domain and therefore the catalytic activity was achieved by specific antibodies (Ossowski et al.,1991), overexpression of the endogenous inhibitors PAI-1 (Ma et al., 1997) and small-molecule inhibitors (Muehlenweg et al., 2001). Using synthetic or naturally occurring inhibitors with low molecular weight is a more cost-effective and feasible way as the use of antibodies and expression of PAI-1 is hampered by poor pharmacokinetics and pharmacodynamics (Su et al., 2015). Using the uPA/serine protease inhibitor Mesupron (WX-671) showed reduced metastatic spread and an extending lifespan in clinical trials of pancreatic and breast cancer patients (Schmitt et al., 2011). To inhibit the downstream actions of the uPA-uPAR complex, another strategy is to block binding of uPA to its receptor. Zhu et al., 2001 could show, that inhibition of uPA-uPAR complex by human uPA-ATF as an antagonist of uPA-uPAR interaction might be a promising anti-invasion and anti-metastasis strategy by displacing uPA from its cognate receptor. uPA-derived cyclic peptides that were synthesized similar to the GFD of uPA that binds to uPAR could show promising results in hampering tumor growth and metastasis in animal models of cancer (Sato et al., 2002). Nucleic acid aptamers are suggested a promising alternative for protease inhibitors. Aptamers recognize their targets with high specificity and affinity corresponding to K_{D} values in the pM to low nM range and therefore being comparable to antibodies. Dupont et al., 2010 were the first one that could identify 2'-F-pyrimidine modified RNA aptamers binding near to the GFD domain and therefore were able to block the binding of uPA to its receptor uPAR with low nM IC₅₀ values. They were able to inhibit the accumulation of uPA-catalyzed plasminogen activation on cell surfaces but none of the aptamers are binding to the serine protease domain and therefore did not block uPA activity. Botkjaer et al. 2012 could show a 2'-fluoro-pyrimidine RNA aptamer that is binding to the catalytic domain of pro-uPA. Due to binding, the proteolytic conversion of pro-uPA to the active two-chain HMW-uPA was delayed, but no inhibition of plasminogen activation could be achieved. Though they could demonstrate that this RNA aptamer also inhibited binding of pro-uPA to uPAR and therefore had significant effects on tumor cell intravasation and dissemination in vivo (Botkjaer et al., 2012). Other DNA aptamers known today are only binding to pro-uPA (Skrypina et al., 2004).

The object of the present invention was thus the provision of new means for treatment of cancer and/or prevention of cancer.

Here, the in vitro selection of single stranded DNA aptamers targeting uPA at different binding epitopes by systematic evolution of ligands by exponential enrichment (SELEX) was presented. DNA aptamers were isolated from a 42-nt random library with a theoretical diversity of 1.9 x 10^25 different species. Binding of aptamers was characterized for affinity and specificity by various assays, including microtiter plate binding assays, electrophoretic mobility shift assay (EMSA), Surface Plasmon Resonance (SPR) spectroscopy and MicroScale Thermophoresis (MST).

### 1 ^{st} aspect - DNA aptamer for use as a medicament

Based thereon, the problem was solved by a DNA aptamer comprising a polynucleotide sequence having at least 70 % identity to a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO: 10 and SEQ ID NO:11 for use as a medicament. Preferably, the DNA aptamer is for use in treatment of cancer and/or prevention of cancer in a subject. In some preferred embodiments, the cancer is bladder cancer or breast cancer.

The aptamers of the invention and combinations thereof, were able to discriminate between HMWand LMW-uPA and therefor binding either to the serine protease domain or to the amino-terminal fragment. Considering also the small size of aptamers together with their polyanionic nature that results in good tissue penetration and rapid blood clearance, they have proven as promising agents in therapeutics. They also showed no immunoreactivity and low to no toxicity and therefore are therapeutics with the potential to inhibit functionally important interactions or the catalytic activity of uPA.

For ease of comparison, the following table 1 shows the SEQ ID Numbers as used in the sequence protocol and aligns these sequences with the names used in following text and in the experimental section.

**Table 1 SEQ ID Numbers as used in the sequence protocol, aligned with the names used in the description and the experimental section.**

| Name^{∗} | SEQ ID No. |
|---|---|
| uPAapt-01 | 1 |
| uPAapt-02 | 2 |
| uPAapt-02-R | 3 |
| uPAapt-02-F | 4 |
| uPAapt-02-FR | 5 |
| uPAapt-03 | 6 |
| uPAapt-06 | 7 |
| uPAapt-08 | 8 |
| uPAapt-21 | 9 |
| uPAapt-26 | 10 |
| uPAapt-27 | 11 |
| uPAapt-0 1-FR | 12 |
| uPAapt-08-FR | 13 |
| uPAapt-27-F | 14 |
| uPAapt-27-R | 15 |
| uPAapt-27-FR | 16 |
| forward primer [5'-Primer] | 17 |
| reverse primer binding site [3'-Primer of forward strain] | 18 |
| pro-uPA^{∗∗} | 19 |
| HMW-uPA A Chain | 20 |
| HMW-uPA B Chain | 21 |
| LMW-uPA (Residue) A Chain | 22 |
| LMW-uPA | 23 |
| B Chain | |
| Con | 24 |

| | |
|---|---|
| ^{∗} "-FR" means the pure core sequence without forward primer and without reverse primer binding site. The forward primer has nucleotide sequence SEQ ID No.15, the reverse primer binding site has nucleotide sequence SEQ ID No.16. If a name is indicated as XXX-F, it means the core sequence together with the reverse primer binding site, but without forward primer; if a name is indicated as XXX-R, it means the core sequence together with the forward primer, but without reverse primer binding site. "XXX" stands for any number of letters and / or digits. ^{∗∗} the sequence of pro-UPA is also listed in the UniProtKB database as ChainⁱPRO_0000028318 of UniProtKB - P00749 [UROK _HUMAN] | |

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one".

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

An "aptamer" is a short, single-stranded DNA (ssDNA) or RNA oligonucleotide with a length of 15-150 bases - in the context of the present invention, preferably a DNA aptamer within the range of from 25 to 90 bases. Unless specifically indicated otherwise herein, the sequences specified, in particular the sequences of the DNA aptamers, may be comprised in larger sequence structures and may be covalently or non-covalently linked to carrier molecules, solid phases, detection/reporter molecules etc.

The DNA aptamer as described above, i.e. the DNA aptamer comprising a polynucleotide sequence having at least 70 % identity to a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO: 11, is able to inhibit binding of Urokinase type plasminogen activator (uPA) with Urokinase type plasminogen activator receptor (uPAR) and optionally able to inhibit activity of Urokinase type plasminogen activator (uPA).

The DNA aptamer comprising a polynucleotide sequence having at least 70 % identity, preferably least 80 % identity, more preferably at least 90 % identity, more preferably at least 95 % identity, more preferably at least 97 % identity, to a sequence selected from the group consisting of SEQ ID NO: 1, SEQ ID NO:3, SEQ ID NO:4, SEQ ID NO:5 and SEQ ID NO:6 is able to inhibit binding of Urokinase type plasminogen activator (uPA) with Urokinase type plasminogen activator receptor (uPAR) and also able to inhibit activity of Urokinase type plasminogen activator (uPA). The DNA aptamer comprising a polynucleotide sequence having at least 70 % identity to a sequence selected from the group consisting of SEQ ID NO:7, SEQ ID NO:8, SEQ ID NO:9, SEQ ID NO:10 and SEQ ID NO:11 is able to (only) inhibit binding of Urokinase type plasminogen activator (uPA) with Urokinase type plasminogen activator receptor (uPAR).

The DNA aptamer is more preferably a polynucleotide, wherein the sequence identity of the first nucleotide sequence is least 80 %, preferably at least 90 %, more preferably at least 95 %, more preferably at least 97 %, and/or wherein the sequence identity of the second sequence is at least 80 %, preferably at least 90 %, more preferably at least 95 %, and/or wherein the sequence identity of the third sequence is at least 80 %, preferably at least 90 %, more preferably at least 95 %.

An identity of xx % means that sequences are comprised, which have a nucleic acid sequence which differs due to at least one substitution, deletion, insertion and/or addition from the indicated sequence. The term "at least one", as used herein means one, or more than one, such as "at least two", "at least three", "at least four", "at least five", etc..

The degree of identity (e.g. expressed as "%identity") between two biological sequences, preferably DNA in the context of the present invention, can be determined by algorithms well known in the art. Preferably, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, preferably over the whole length of the polynucleotide or polypeptide, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used.

The DNA aptamer of the present invention is used as it is or in a stabilized form or, as a person skilled in the art is aware of, in a functionalized form.

"Stabilization": For example, in view of improved stability of the oligonucleotides against enzymatic degradation, e.g. by nucleases, one or more nucleoside residues can be coupled to others by any of the numerous known internucleoside linkages, preferably selected from the group consisting of phosphodiester, phosphorothioate, phosphorodithioate, alkylphosphonate, alkylphosphonothioate, phosphotriester, phosphoramidate, siloxane, carbonate, carboalkoxy, acetamidate, carbamate, morpholino, borano, thioether, bridged phosphoramidate, bridged methylene phosphonate, bridged phosphorothioate, and sulfone internucleoside linkages. The aptamer of the present invention may also have modified nucleosides, for example the sugar may have a substituent in 2' position selected from the group consisting of hydroxyl group, methoxy group, fluorine atom, and amino group, the 2' oxygen and the 4' carbon of the sugar may be bridged by a -CH₂-O- bond (LNA). The aptamer of the present invention may also have modified bases, for example, the base may carry a substituent so that the aptamers off-rate is slowed (slow off-rate modified aptamers, SOMAmers) at the C5-postion of pyrimidines (dU/dC) and/or may be modified via click chemistry (Clickmers) for example at a C5-position of a 5-ethyhl-2'-deoxyuridine (EdU) [see Plückthun et al., 2020]. The aptamer of the invention may also be conjugated to a carrier molecule and/or to a reporter molecule. Carrier molecules comprise such molecules that, when conjugated to the aptamer, prolong the plasma half-life of the conjugated aptamer, e.g. by enhancing the stability and/or by affecting the excretion rate. Preferably, suitable carrier molecules are selected from the group consisting of polyethylene glycol (PEG), cholesterol, (cholesteryl-aptamer), diacylglycerol (DAG conjugation) and N-acetyl glucosamine (OGlcNAc conjugation), which can be conjugated to the 5' and/or the 3' end of the aptamer. Reporter molecules comprise molecules that allow for the detection of the conjugated aptamer. Examples of such reporter molecules are horseradish peroxidase (HRP), green fluorescent protein (GFP), biotin, HLP, dyes like e.g. fluorescence dyes, for example, cyanine *5 (Cy5)* or coumarin 6 *(C6),* electrochemically active reporter molecules and/or compounds. The aptamer may also have a 3' capping, for example, inverted thymidine (inverted dT) can be bound to the 3' end of the aptamer. It is also feasible to use a modification with D-/L- isonucleotide(s) and/or L-nucleotide(s). The aptamer may also be used in circular form with a 3' - 5' termini linkage. Further, the aptamer may be encapsulated in suitable vehicles to protect their structural integrity as well as to promote their delivery inside cells. Preferred vehicles include liposomes, lipid vesicles, and microparticles.

"Functionalization" means any type of chemical modification, which is usable for example, for coupling with, for example, reporter molecules or small-molecule drugs resulting in aptamer-drugconjugates. Additionally, functionalization can comprise chemical groups for aptamer surface immobilization, for example on nanoparticle, for example, an amino (-NH₂) group, a thiol (-SH) group, a carboxyl group such as -COOH and -COOR respectively, wherein R represents, for example, a C1 to C10 alkyl group; a biotin group, a dibenzocyclooctyne (-DBCO) group, wherein the group(s) is/are present for example, at the 5'- and/or 3'-end of the aptamer.

The term "inhibition of binding of Urokinase type plasminogen activator (uPA) with Urokinase type plasminogen activator receptor (uPAR)" is, in principle, understood by the skilled person to relate to any modulation causing the binding of Urokinase type plasminogen activator (uPA) with Urokinase type plasminogen activator receptor (uPAR) to decrease. As is understood by the skilled person, said modulation may be achieved directly, e.g. by preventing interaction of Urokinase type plasminogen activator (uPA) with Urokinase type plasminogen activator receptor, or by reducing the amount of uPA and/or uPAR. Methods for determining inhibition of binding of Urokinase type plasminogen activator (uPA) with Urokinase type plasminogen activator receptor (uPAR) are known in the art and are shown -exemplarily- herein in the Examples. The term "inhibition of activity of Urokinase type plasminogen activator (uPA)" is, in principle, understood by the skilled person to relate to any modulation causing the activity of Urokinase type plasminogen activator (uPA) to decrease. As is understood by the skilled person, said modulation may be achieved, for example, by blocking the catalytic domain of uPA. Methods for determining inhibition of activity of Urokinase type plasminogen activator (uPA) are known in the art and are shown -exemplarilyherein in the Examples. As is understood by the skilled person, the term "inhibition" includes partial inhibition. Thus, in the case of a quantifiable event, inhibition preferably is a reduction of said event by at least 25%, more preferably at least 50%, still more preferably at least 75%, most preferably at least 85% when a DNA aptamer as described above is applied, preferably compared to a control, wherein no DNA aptamer as described above is applied. Partial inhibition thus also comprises a slowing of the conversion of plasminogen into plasmin by uPA, i.e. the conversion is at least 25%, more preferably at least 50%, still more preferably at least 75%, most preferably at least 85%, slower when a DNA aptamer as described above is applied, preferably compared to a control, wherein no DNA aptamer as described above is applied.

The term "subject" as used herein refers to any kind of animal encompassing, e.g., mammals, birds, fish or reptiles. Typically, the animal, however, is a mammal such as a mammals used as pets including dogs, cats, horses, or rodents, laboratory animals, e.g., rats, mice or apes, or farming animals such as pigs, cows, goats, or sheep. More preferably, the mammal is a primate and, more preferably, a human. Preferably, the subject is known or suspected to suffer or have suffered from cancer. Also preferably, the subject was identified as a subject suffering from cancer according to the method as specified herein below in the third aspect.

### 2^{nd} aspect - DNA aptamer

In a second aspect, the invention relates to a DNA aptamer comprising a polynucleotide sequence having at least 70 % identity to a sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11. Preferably, the sequence identity is least 80 %, more preferably at least 90 %, more preferably at least 95 %, more preferably at least 97 %. All details described above in the section related to the first aspect also apply for the second aspect as well. This includes, inter alia, the ability to inhibit binding of Urokinase type plasminogen activator (uPA) with Urokinase type plasminogen activator receptor (uPAR) and optionally able to inhibit activity of Urokinase type plasminogen activator (uPA) and that especially the DNA aptamer comprising a polynucleotide sequence having at least 70 % identity, preferably least 80 % identity, more preferably at least 90 % identity, more preferably at least 95 % identity, more preferably at least 97 % identity, to a sequence selected from the group consisting of SEQ ID NO: 1, SEQ ID NO:3, SEQ ID NO:4, SEQ ID NO:5 and SEQ ID NO:6 is able to inhibit binding of Urokinase type plasminogen activator (uPA) with Urokinase type plasminogen activator receptor (uPAR) and also able to inhibit activity of Urokinase type plasminogen activator (uPA). The DNA aptamer comprising a polynucleotide sequence having at least 70 % identity to a sequence selected from the group consisting of SEQ ID NO:7, SEQ ID NO:8, SEQ ID NO:9, SEQ ID NO:10 and SEQ ID NO:11 is able to (only) inhibit binding of Urokinase type plasminogen activator (uPA) with Urokinase type plasminogen activator receptor (uPAR). Also included, as outlined in detail in the section related to the first aspect, is the usability as a medicament. Preferably, the DNA aptamer is usable in treatment of cancer and/or prevention of cancer in a subject, wherein the subject is most preferably a human. The cancer is preferably bladder cancer or breast cancer.

### 3^{rd} aspect - method for identifying a subject benefiting from cancer treatment with a DNA aptamer

A third aspect of the invention is related to a method for identifying a subject benefiting from cancer treatment with a DNA aptamer comprising:
(a) contacting a sample comprising cancer cells of said subject with a DNA aptamer comprising a polynucleotide sequence having at least 70 % identity to a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11;
(b) determining the degree of inhibition of binding of Urokinase type plasminogen activator (uPA) with Urokinase type plasminogen activator receptor (uPAR) and/or the degree of inhibition of Urokinase type plasminogen activator (uPA) in the cancer cells of step (a),
(c) comparing the degree of inhibition determined in step (b) to a reference, preferably from control treated cancer cells of said subject or from untreated cells of a healthy subject, and
(d) based on the result of step (c), identifying a subject benefiting from treatment with a polynucleotide having at least 70 % identity to a sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11.

Preferably, the sequence identity is least 80 %, more preferably at least 90 %, more preferably at least 95 %, more preferably at least 97 %. All details described above in the section related to the first aspect also apply for the second aspect as well.

Preferably, the reference is derived from control treated cancer cells of said subject, i.e., preferably, cancer cells of said subject not treated with an DNA aptamer as described above, but, more preferably, otherwise treated in an identical manner; if, in such case, a threshold value is defined based on said control treated cancer cells, a subject benefiting from treatment with DNA aptamer as described above is identified if the degree determined in step (c) is higher than said reference.

The method for identifying a subject benefiting from cancer treatment with a DNA aptamer is preferably an *ex vivo* or *in vitro* method, more preferably an *in vitro* method.

Moreover, the method may comprise steps in addition to those explicitly mentioned above. For example, further steps may relate, e.g., to providing a sample for step a), or determining additional markers in step b). Moreover, one or more of said steps may be performed by automated equipment. Also, the method for identifying a subject benefiting from treatment with a polynucleotide may be part of a method of treatment comprising first identifying a subject benefiting from treatment with a polynucleotide and then treating said subject with a polynucleotide, preferably as specified herein below.

The term "contacting" is understood by the skilled person. Preferably, the term relates to bringing a compound as specified into physical contact with a sample or with a further compound and thereby, e.g. allowing the sample and the compound to interact.

The term "sample", as used herein, relates to a sample of a body fluid, a sample from a tissue or an organ, or a sample of wash/rinse fluid or a swab or smear obtained from an outer or inner body surface, said sample being known or suspected to comprise leukemia cells. Preferably, the sample is a blood, plasma, serum, urine, saliva, or lacrimal fluid sample. Samples can be obtained by use of brushes, (cotton) swabs, spatula, rinse/wash fluids, punch biopsy devices, puncture of cavities with needles or lancets, or by surgical instrumentation. However, samples obtained by well-known techniques including, in an embodiment, scrapes, swabs or biopsies from the urogenital tract, perianal regions, anal canal, the oral cavity, the upper aerodigestive tract and the epidermis are also included. Preferably, samples are obtained from a tissue or an organ or a body fluid known to comprise cancer cells if present in a subject, i.e., preferably, in case of bladder cancer, the sample is preferably a tissue sample (from biopsies) or an urine sample or a serum sample, in case of breast cancer, the sample is preferably a tissue sample (from biopsies) or a blood sample or a serum sample. It is to be understood that the sample may be further processed in order to carry out the method of the present invention. Particularly, cells may be obtained from the sample by methods and means known in the art. Thus, the term sample also may relate to preparations comprising or suspected to comprise leukemia cells, diluted, enriched, purified and/or cultivated from a sample.

Also, the skilled person is able to provide appropriate references. The reference may be a threshold vale, a reference range, a score including the amount(s) of the marker(s) determined as a parameter, or any other reference deemed appropriate by the skilled person. For establishing a score, other parameters may additionally be included as parameters, e.g. cancer stage, histological parameters such as degree of differentiation of cancer cells, risk factors associated with the subject, and/or results of genetic assessments, e.g. evaluation of chromosome aberrations in cancer cells of the subject. Preferably, the reference is derived from control treated cancer cells of the same subject or from a population of apparently healthy subjects. Also preferably, the reference is derived from control treated cancer cells of said subject, i.e., preferably, cancer cells of said subject not treated with a polynucleotide as described above, but, more preferably, otherwise treated in an identical manner; if, in such case, a threshold value is defined based on said control treated cancer cells, a subject benefiting from treatment with a polynucleotide is identified if the amount determined in step (b) is higher than said reference. Also preferably, the reference is derived from a population of healthy subjects; if, in such case, a threshold value is defined based on said population, a subject benefiting from treatment is identified if the amount determined in step (b) is equal to or higher than the reference.

### 4^{th} aspect - kit

In a fourth aspect, the invention relates to a kit, preferably a kit comprising (i) a DNA aptamer comprising a polynucleotide having at least 70 % identity to a sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO: 10 and SEQ ID NO:11 and (ii) a means for determining inhibition of binding of Urokinase type plasminogen activator (uPA) with Urokinase type plasminogen activator receptor (uPAR) and/or a means for determining an inhibition of Urokinase type plasminogen activator (uPA) in a sample. Preferably, the kit is comprised in a housing. The kit preferably further comprises a diluent and/or a means of administration. Preferably, the sequence identity is least 80 %, more preferably at least 90 %, more preferably at least 95 %, more preferably at least 97 %. All details described above in the section related to the first aspect also apply for the second aspect as well.

In view of the above, the following embodiments are particularly envisaged:
1. DNA aptamer comprising a polynucleotide sequence having at least 70% identity to a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11 for use as a medicament.
2. The DNA aptamer for use according to embodiment 1 in treatment of cancer and/or prevention of cancer in a subj ect.
3. The DNA aptamer for use according to embodiment 1 or 2, which is able to inhibit binding of Urokinase type plasminogen activator (uPA) with Urokinase type plasminogen activator receptor (uPAR) and optionally able to inhibit activity of Urokinase type plasminogen activator (uPA).
4. The DNA aptamer for use according to any one of embodiments 1 to 3 comprising a polynucleotide sequence having at least 70 % identity to a sequence selected from the group consisting of SEQ ID NO: 1, SEQ ID NO:3, SEQ ID NO:4, SEQ ID NO:5 and SEQ ID NO:6.
5. The DNA aptamer for use according to any one of embodiments 1 to 4, wherein the sequence identity is least 80 %, preferably at least 90 %, more preferably at least 95 %, more preferably at least 97 %.
6. The DNA aptamer for use according to any one of embodiments 2 to 5, wherein the cancer is bladder cancer or breast cancer.
7. The DNA aptamer for use according to any one of embodiments 1 to 6, wherein the subject is a human.
8. A DNA aptamer comprising a polynucleotide sequence having at least 70 % identity to a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11.
9. The DNA aptamer according to embodiment 8, wherein the sequence identity is least 80 %, preferably at least 90 %, more preferably at least 95 %, more preferably at least 97 %.
10. A method for identifying a subject benefiting from cancer treatment with a DNA aptamer comprising:
   (a) contacting a sample comprising cancer cells of said subject with a DNA aptamer comprising a polynucleotide sequence having at least 70 % identity to a sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11;
   (b) determining the degree of inhibition of binding of Urokinase type plasminogen activator (uPA) with Urokinase type plasminogen activator receptor (uPAR) and/or the degree of inhibition of Urokinase type plasminogen activator (uPA) in the cancer cells of step (a),
   (c) comparing the degree of inhibition determined in step (b) to a reference, preferably from control treated cancer cells of said subject, and
   (d) based on the result of step (c), identifying a subject benefiting from treatment with a polynucleotide having at least 70 % identity to a sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11.
11. The method according to embodiment 10, which is an *ex vivo* or *in vitro* method, preferably an *in vitro* method.
12. A kit comprising (i) a DNA aptamer comprising a polynucleotide having at least 70 % identity to a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11 and (ii) a means for determining inhibition of binding of Urokinase type plasminogen activator (uPA) with Urokinase type plasminogen activator receptor (uPAR) and/or a means for determining an inhibition of Urokinase type plasminogen activator (uPA) in a sample.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

### Examples

### Materials and Methods

### Target preparation

Native high-molecular weight (HMW-uPA) isolated from human urine was purchased from ProSpec-Tany TechnoGene Ltd. (Ness-Ziona, Israel). To select Aptamers against both uPA forms, the low-molecular weight form of uPA (LMW-uPA) was obtained by autocatalytic conversion (Nobuhara et al., 1981). Therefore, HMW-uPA was dissolved in PBS to a concentration of 1.0 µg/µl and incubated at 37 °C for a minimum of 32 days. To obtain the 33 kDa LMW-uPA the sample was purified and concentrated by a Nanosep^{®} Centrigual Device with Omega^{™} Membrane 30 K (Pall Corporation, United States). Complete conversion and purity of LMW-uPA was determined by SDS-gel electrophoresis. For further use, both uPA forms were biotinylated separately using EZ-Link Sulfo-NHS-SS-Biotin (Thermo Fisher Scientific Inc., Germany) according to manufacturer instructions.

### Oligonucleotides and semiautomatic in vitro selection

The used single stranded DNA oligonucleotide library, whose theoretical diversity is over 10²⁵ was used as a precursor pool for aptamer selection. A 42-nt random sequence was flanked by 20-nt constant primer regions on each side (5'-AGGTAGAGGAGCAAGCCATC-(N₄₂)-GATGCGTGATCGAACCTACC-3'), the *forward* primer [5'-Primer] and the *reverse* primer binding site [3'-Primer of forward strain]. Selection of aptamers binding uPA was performed using a semiautomatic selection process as described by Wochner et al., 2007. Before selection, the single-stranded DNA library was heated up to 90 °C for 3 min and slowly (for about 30 min) cooled down to 25 °C in binding buffer BPs-T (50 mM Bis-Tris/HCl pH 6.5, 110 mM NaCl, 5 mM MgCl2, 1 mM CaCl2, 1 mM KCl, 0,05 % v/v Tween^{®} 20). A mixture from three different providers of an 82-nt single-stranded DNA library was used for the first selection round (Noxxon Pharma AG, Metabion GmbH, IBA GmbH; Germany). To select aptamers against HMW- and LMW-uPA, selection was carried out separately for each uPA form. Therefore, the renatured DNA was incubated separately with the biotinylated uPA forms for 1 h at room temperature in binding buffer BPs-T. After incubation, each biotinylated uPA form with bound DNA was coupled to streptavidin-coated magnetic beads (Dynabeads M-280 streptavidin, Dynal, Norway) to transfer them automatically for several washing steps. Remaining biotin binding sites were blocked with 20 µM biotin in BPs-T. To reduce the selection of matrix binders, preselection steps with biotin blocked streptavidin beads as well as uncoupled streptavidin beads were carried out prior to uPA incubation. Extensive washing steps with the same buffer were performed after coupling to streptavidin-coated beads to remove non-binding oligonucleotides. DNA-sequences binding to uPA were eluted unspecifically by 8M urea in BPs-T for 30 min at 65 °C and 900 rpm in a thermoshaker. For an additional specific elution in some selection rounds the disulfide bond of the EZ-Link Sulfo-NHS-SS-Biotin linked to the uPA-forms was reduced by 500 mM dithiothreitol (DTT) in BPs-T for 60 min prior to unspecific elution. Eluted fractions were precipitated with 2-propanol at -20 °C over night. Precipitaed DNA was amplified by PCR with 5'-AGGTAGAGGAGCAAGCCATC-3' used as the *forward* primer, and 5'-biotin-C6-GGTAGGTTCGATCACGCATC-3' used as the *reverse* primer, which allowed the subsequent purification of the aptamer strand by alkali denaturation on streptavidin magnetic beads. After 11 selection rounds for HMW-uPA and 12 selection rounds for LMW-uPA, enriched DNA-pools with high-affinity target binders was identified for both uPA-forms by fluorescent dye-linked aptamer assay (FLAA). Enriched DNA-pools were sequenced using next generation sequencing (NGS) technique by GATC Biotech (Ebersbach, Germany) and following sequence analysis was performed with the COMPAS analysis software by AptaIT (München, Germany).

### uPA inhibition assay

Inhibition of uPA activity was tested by the Chemicon PAI Activity Assay Kit (Merck KGaA, Germany) according to manufacturer's specifications with some modifications. In this case 10× BPs-T (500 nM Bis-Tris/HCl pH 6.5, 1.1 NaCl, 50 mM MgCl₂, 10 mM CaCl₂, 10 mM KCl, 0.5 % v/v Tween^{®} 20) was used as the assay buffer (15 µl for the aptamer samples and 20 µl for control samples). Individual aptamers were tested in a fivefold excess for inhibition of enzyme activity to obtain a ratio (aptamer:target) of 5:1. For each well 600 pmol of aptamer were renatured in 50 µl BPs-T by heating up to 90 °C for 3 min and slowly cooling down to 25 °C separately. 10 µl 0.65 µg/µl uPA from the assay kit (corresponds to 120 pmol) alone or in the presence of individual renatured aptamers were preincubated for 30 min at 23 °C and 350 rpm in a microtiterplate. Double-destilled-water (ddH₂O) was added for each sample to end up to a total volume of 180 µl before preincubation. For the control (uPA alone), aptamer solution was replaced by 50 µl of ddH₂O. For the sample with no uPA the 10 µl of uPA was replaced by 10 µl ddH₂O. Activated plasminogen activator inhibitor (PAI-1) was prepared as described by the manufactures protocol and was used as a positive control. Here, the aptamer solution was replaced by 40 µl of activated PAI-1 solution and 10 µl ddH₂O. For the test of inhibition by uPAapt-02-FR at various concentrations over the time, a serial dilution of renatured uPAapt-02-FR from 185 µM to 0.36 µM was prepared in BPs-T and 50 µl of each dilution (corresponds to 9,250 - 18 pmol) were preincubated with 10 µl 0.5 µg/µl uPA from the assay kit (corresponds to 92.5 pmol) as described previously. Afterwards 20 µl of the chromogenic substrate (2.5 µg/µl tripeptide with pNA group) was added and hydrolysis was monitored by measuring the absorbance at 405 nm was measured by EnVision^{®} 2105 multimode plate reader after incubation at 37 °C for 4 hours or every two minutes at 37 °C over the period of 260 minutes. Samples were tested in duplicates.

### Surface Plasmon Resonance for uPA-uPAR interaction

Surface plasmon resonance (SPR) experiments were carried out using a Biacore T200 (GE Healthcare/Biacore, Sweden). To determine the uPA-uPAR inhibitory activity of the aptamers, 5 µg/ml human uPAR (Human uPAR / CD87 Protein, Sino Biological Inc., USA; UniProtKB database Q03405 (UPAR _HUMAN)) was prepared in 10 mM NaOAc pH 5.0 and immobilized on an EDC/NHS-activated HC200M SPR Sensor Chip (Xantec, Germany) to a level of approximately 500 RU using Biacore immobilization wizard. The system was operating at 25 °C and preparation of the sensor chip was performed at a flow rate of 10 µl/min. The reference surface as well as remained binding sites were blocked with 1 M ethanolamine for 420 sec. Human uPA alone (20 nM) or together with aptamers in different concentrations (3.9 - 2000 nM) were prepared in BPs-T and passed over the sensor surfaces at a flow rate of 30 µl/min for 90 s and a 180 s delay. Herein, HMW-uPA was used as uPA. Aptamers alone (3.9 - 2000 nM) were injected as well to exclude binding of aptamers to uPAR. Before working with the aptamers, they were renatured as described previously. The chip surface was regenerated with of 100 mM acetic acid (pH 2.8) containing 0.5 M NaCl for 15 s at a flow rate of 20 µl/min. Before determining IC₅₀-values, all aptamers were screened for the uPA-uPAR inhibitory activity by injecting uPA alone (20 nM) or together with individual aptamers (500 nM) over the sensor surface. Binding of aptamers to uPAR was tested by injecting aptamers alone (500 nM). Aptamers that showed the best results for inhibition of uPA binding to uPAR were selected for IC₅₀ calculation. The response of uPA in complex with different concentrations of each aptamer were plotted as a function of aptamer concentrations relative to the response of uPA without aptamer. As repeated regeneration steps were conducted, binding of uPA to the sensor surface immobilized with uPAR reduces over the course of measurements. To include this in IC₅₀ calculation, uPA was injected before each concentration series of the different aptamers and the loss of the signal was fitted using a polynomial regression model. Each uPA-aptamer-complex response was reduced by the calculated loss of signal. IC₅₀-values were estimated by nonlinear regression analysis, fitting the data to a dose response function using OriginPro 2021 (OriginPro 2021 Software, OriginLab Corporation, USA). A sequence-unrelated control oligonucleotide (Con) was analyzed for comparison and to demonstrate sequence dependent inhibition. Two independent experiments were performed. The mean value of the relative response with the respective standard deviation were used for IC₅₀ value determination.

### Results

### DNA aptamers

All DNA aptamers investigated are shown with their sequences in Table 2 and are named as uPA aptamers (uPAapt-xx). The naming and the SEQ ID Numbers
used in Table 2 is the same as used above in Table 1.

**Table 2 Aptamer sequences, represented in 5'-3' direction. The 41-42 nt long variable region (shown free from any underlining) is flanked by the 20 nt long forward- primer (5'-Primer) and reverse-primer binding site (3'-Primer of forward strain) shown with simple (forward) or double (reverse) underlining).**

| Name | Nucleotide Sequence (5'-3') | SEQ ID No. |
|---|---|---|
| uPAapt-0 1 | AGGTAGAGGAGCAAGCCATC ATGGTAACATGCACTAGGTCGCGATGGTTCCGTCTGATCGCT GATGCGTGATCGAACCTACC | 1 |
| uPAapt-02 | AGGTAGAGGAGCAAGCCATC CAAGCGGGGGTGAGAGATCTGTCAGTACGAGCTGGGTTTGCG GATGCGTGATCGAACCTACC | 2 |
| uPAapt-02-R | AGGTAGAGGAGCAAGCCATC CAAGCGGGGGTGAGAGATCTGTCAGTACGAGCTGGGTTTGCG | 3 |
| uPAapt-02-F | CAAGCGGGGGTGAGAGATCTGTCAGTACGAGCTGGGTTTGCG GATGCGTGATCGAACCTACC | 4 |
| uPAapt-02-FR | CAAGCGGGGGTGAGAGATCTGTCAGTACGAGCTGGGTTTGCG | 5 |
| uPAapt-03 | AGGTAGAGGAGCAAGCCATC ACGGAGGCAGAGGGTGAGAGATCTGTTAGTACTAGCCCATGT GATGCGTGATCGAACCTACC | 6 |
| uPAapt-06 | AGGTAGAGGAGCAAGCCATC GGGTGGGTGGGTGGGTGATGGCTCGACTCTACCCTGGCGCTG GATGCGTGATCGAACCTACC | 7 |
| uPAapt-08 | AGGTAGAGGAGCAAGCCATC CAGCGGTAGGGGTTATATAGCTGCGCCATAGGGTACTCGTG GATGCGTGATCGAACCTACC | 8 |
| uPAapt-21 | AGGTAGAGGAGCAAGCCATC GGAGGTACTCACCGACGCTGAACTCCATAGAATGTGGTGATG GATGCGTGATCGAACCTACC | 9 |
| uPAapt-26 | AGGTAGAGGAGCAAGCCATC ATCCGTGGCGTGGTTGGTGGGGGAGGTGGGCGGGTAGACGGT GATGCGTGATCGAACCTACC | 10 |
| uPAapt-27 | AGGTAGAGGAGCAAGCCATC ATGGGGTGTTATGGGGGGGGCTTATTGCGGTCAGGGGACGAT GATGCGTGATCGAACCTACC | 11 |
| uPAapt-01 - FR | ATGGTAACATGCACTAGGTCGCGATGGTTCCGTCTGATCGCT | 12 |
| uPAapt-08-FR | CAGCGGTAGGGGTTATATAGCTGCGCCATAGGGTACTCGTG | 13 |
| uPAapt-27-F | ATGGGGTGTTATGGGGGGGCTTATTGCGGTCAGGGACGAT GATGCGTGATCGAACCTACC | 14 |
| uPAapt-27-R | AGGTAGAGGAGCAAGCCATC ATGGGGTGTTATGGGGGGGGCTTATTGCGGTCAGGGGACGAT | 15 |
| uPAapt-27-FR | ATGGGGTGTTATGGGGGGGGCTTATTGCGGTCAGGGGACGAT | 16 |
| Con | GGGAATTCGAGCTCGGTACCGTACAGTACTGCATATCTCATA CTTCCTAGATACCATCCCTGCAGGCATGCAAGCTTGG | 24 |

### Inhibition of proteolytic activity of uPA

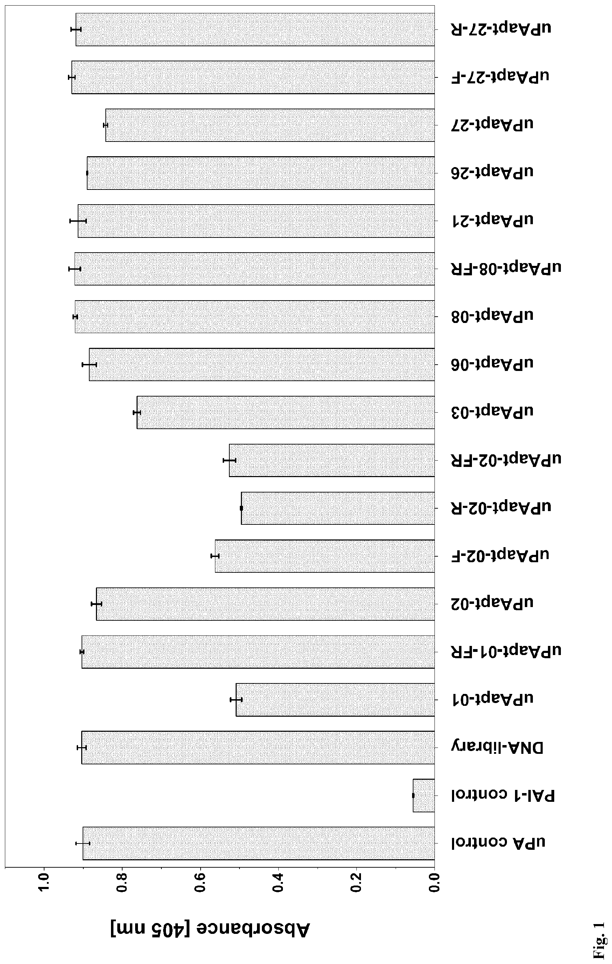

While uPAapt-01, uPAapt-02-F, uPAapt-02-R, uPAapt-02-FR and uPAapt-03 were able to inhibit the catalytic activity of uPA by at least 10%, while uPAapt-02 and uPAapt-27 showed almost no effect on the activity of uPA, wherein "almost no effect" means an inhibition of < 10%. Especially uPAapt-01, uPAapt-02-R, uPAapt-02-F, uPAapt-02-FR were able to inhibit the catalytic activity of uPA, i.e. to reduce the catalytic activity of uPA, by at least 35% (uPAapt-01: 44 %, uPAapt-02: 4%, uPAapt-02-F: 38%, uPAapt-02-R: 45% , uPAapt-02-FR: 42%, uPAapt-03: 15%, uPAapt-27: 6%). The calculation was made based on uPA without aptamer having 0% uPA inhibition. All other aptamers, which are assumed to bind in other regions of uPA, showed no effect (Fig. 1).

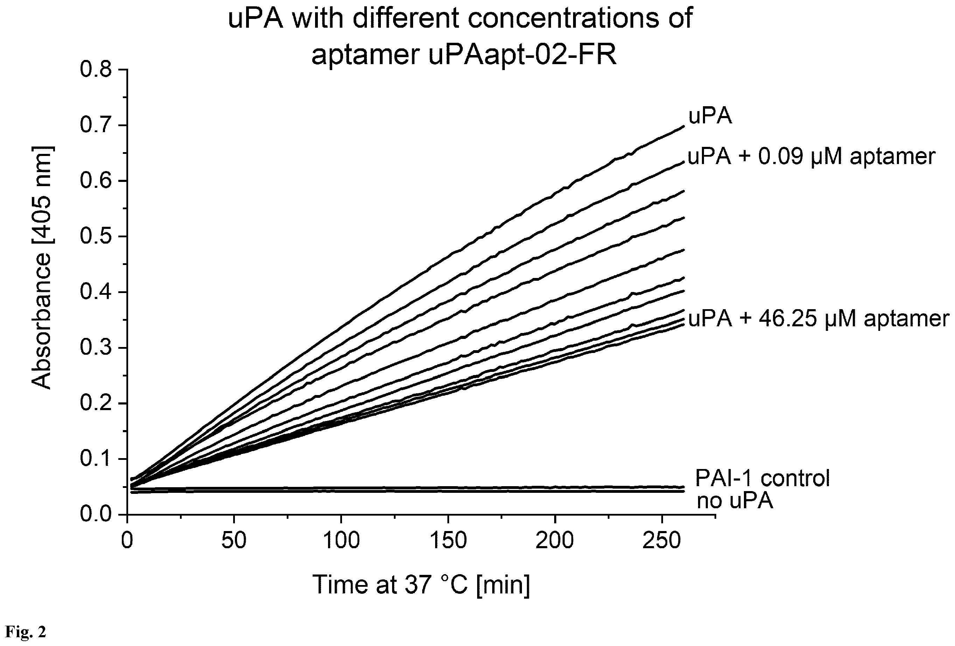

As several aptamers showed similar inhibitory effects, uPAapt-02-FR was selected for further characterization. As a representative aptamer, uPAapt02-FR showed a dose dependent effect on the inhibition of uPA activity - uPA was used in a concentration of 0.46 µM - with a substantial reduction in the conversion of the chromogenic peptide substrate in the range of 0.09 µM to 46.25 µM. Here, the highest concentration of uPAapt-02-FR (46.25 µM) corresponds to 100-fold excess of aptamer (Fig. 2).

### uPA-uPAR inhibitory activity of aptamers

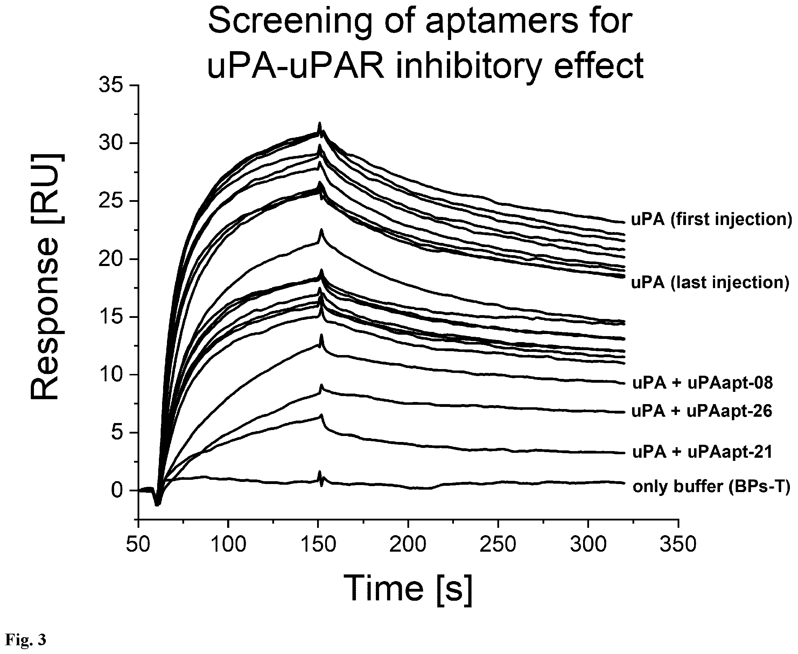

All aptamers were screened for their ability to inhibit uPA-uPAR binding, where several aptamers were able to inhibit binding of uPA to uPAR. Although they differ in strength of inhibition, the following aptamers showed an inhibitory effect: uPAapt-21, uPAapt-26, uPAapt-08, uPAapt-02, uPAapt-01, uPAapt-02-R, uPAapt-02-F, uPAapt-02-FR, uPAapt-03, uPAapt-27 and uPAapt-06. Besides some aptamers which showed no inhibitory effect (uPAapt-01-FR, uPAapt-08-FR, uPAapt-27-FR, uPAapt-27-R, uPAapt-27-F), the DNA library used for aptamer selection as well as the sequence-unrelated control aptamer Con showed also no inhibitory effect. The aptamers that showed the best inhibitory activity, i.e. uPAapt-21, uPAapt-26 and uPAapt-08 (Fig. 3), were used for IC₅₀ calculation (see Table 3).

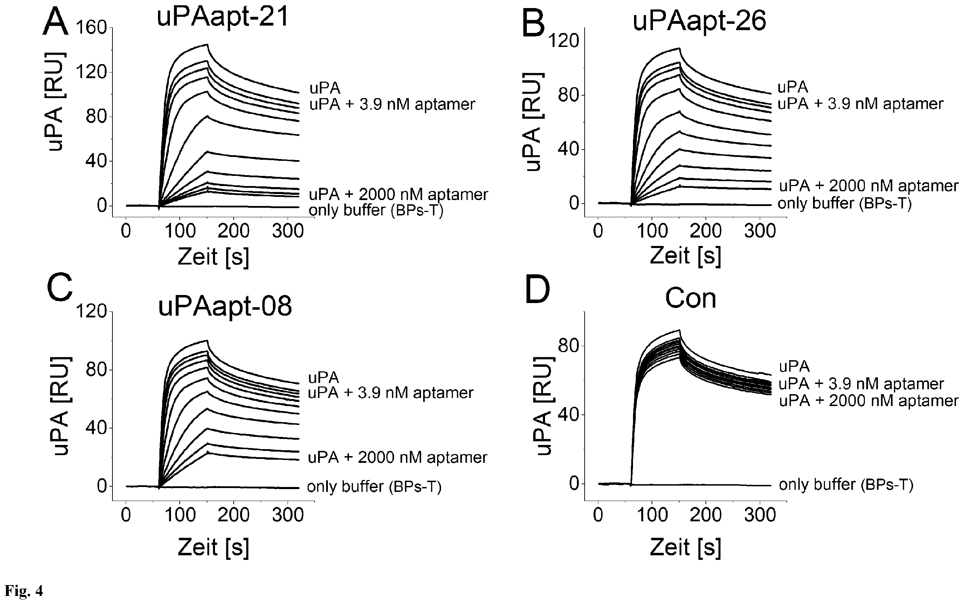

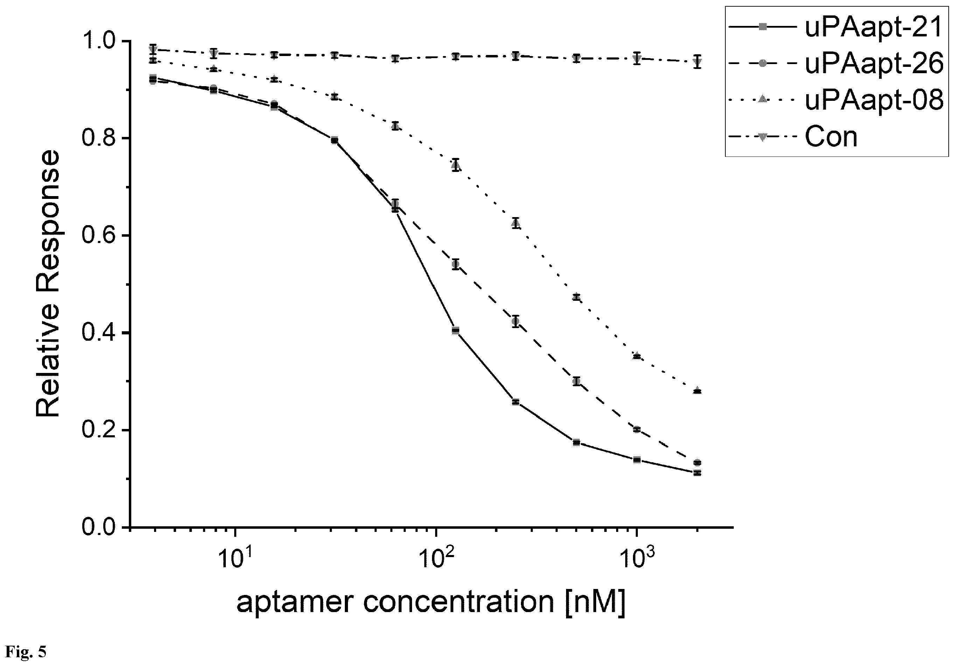

Binding of uPA to the immobilized uPAR on the sensor surface in the presence of increasing concentrations of the different aptamers are shown in Fig. 4. Where uPAapt-21, uPAapt-26 and uPAapt-08 showed a dose dependent effect on the inhibition of uPA binding to uPAR on the sensor surface, as already mentioned, the sequence-unrelated control aptamer Con showed no inhibitory activity. The slight decrease in the reported response values for Con can be attributed to the fact that the ability of uPA binding to uPAR decreases due to the regenerations between each sample injection. No binding of aptamers to uPAR could be detected in both experiments (data not shown). The response of uPA in complex with different concentrations of each aptamer were plotted as a function of aptamer concentrations relative to the response of uPA without aptamer (Fig. 5). As repeated regeneration steps were conducted, binding of uPA to the sensor surface immobilized with uPAR reduces over the course of measurements. To include this in IC₅₀ calculation, uPA was injected before each concentration series of the different aptamers and loss of signal was fitted using a polynomial regression model. Each uPA-aptamer-complex response was reduced by the calculated loss of signal. Based on this data, IC₅₀-values were estimated by a nonlinear regression analysis, fitting the data to a dose response function. IC₅₀-values with its standard error for the inhibition of uPA-uPAR binding by the different aptamers are shown in Table 3.

**Table 3 IC₅₀ values [nM] of selected aptamers for inhibition of interaction between uPA and its receptor uPAR. Values were estimated by nonlinear regression analysis, fitting the data to a dose response function.**

| **Aptamer** | **IC₅₀ (± standard error) [nM]** |
|---|---|
| uPAapt-21 | 87 **(±** 4) |
| uPAapt-26 | 165 **(±** 16) |
| uPAapt-08 | 298 **(±** 17) |
| Con | - |

Overall, it could be shown that especially uPAapt-01, uPAapt-02, uPAapt-02-R, uPAapt-02-F, uPAapt-02-FR, uPAapt-03, uPAapt-06, uPAapt-08, uPAapt-21, uPAapt-26, and uPAapt-27 were able to inhibit binding of Urokinase type plasminogen activator (uPA) with Urokinase type plasminogen activator receptor (uPAR) and optionally able to inhibit activity of Urokinase type plasminogen activator (uPA). Thereof, uPAapt-01, uPAapt-02-R, uPAapt-02-F, uPAapt-02-FR, uPAapt-03 were able to inhibit binding of Urokinase type plasminogen activator (uPA) with Urokinase type plasminogen activator receptor (uPAR) and were also able to inhibit activity of Urokinase type plasminogen activator (uPA). uPAapt-02, uPAapt-06, uPAapt-08, uPAapt-21, uPAapt-26, and uPAapt-27 were able to (only) inhibit binding of Urokinase type plasminogen activator (uPA) with Urokinase type plasminogen activator receptor (uPAR).

### Description of Figures

- Fig. 1: uPA inhibition assay for various aptamers with a molar ratio aptamer:uPA of 5:1. uPA activity measured by the hydrolysis of the chromogenic substrate by measuring the absorbance at 405 nm after 4 hours at 37 °C for uPA (120 pmol) alone or incubated with various aptamers (600 pmol) in a reaction volume of 200 µl . Incubation with PAI-1 or the DNA-library used for aptamer selection were carried as controls in this experiment (number of records N=2).
- Fig. 2:: uPA inhibition by uPAapt-02-FR with a molar ratio aptamer:uPA in the range of from 0.2:1 to 100:1. uPA activity measured by the hydrolysis of the chromogenic substrate by measuring the absorbance at 405 nm over the period of 260 minutes at 37 °C in the presence of increasing concentrations of uPAapt-02-FR (0.09, 0.18, 0.36, 0.72, 1.45, 2.89, 5.78, 11.56, 46.25 µM). While samples added with PAI-1 served as a positive control, uPA (0.46 µM) alone or samples containing no uPA served as negative controls. The total reaction volume was 200 µl (number of records N=2).
- Fig. 3: Screening of aptamers inhibiting uPA-uPAR binding. SPR sensograms showing each response unit for 20 nM human uPA alone or together with individual aptamers (500nM) injected from seconds 60 to 150 on a sensor surface with immobilized human uPAR. As the ability of uPA binding uPAR decreases due to the regeneration between each sample injection, the binding of uPA alone was checked after each four aptamers.
- Fig. 4: Inhibition of uPA binding to uPAR by selected aptamers. SPR sensograms showing the capture of 20 nM human uPA (injected from seconds 60 to 150) on a sensor surface with immobilized human uPAR in the presence of increasing concentrations of aptamers. The sensograms showing each response unit [RU] over the time [s] for uPA alone or in complex with different aptamer-concentrations (2.000, 1000, 500, 250, 125, 62.5, 31.3, 15.6, 7.8 and 3.9 nM). (A) uPAapt-21 (B) uPAapt-26 (C) uPAapt-08 (D) Con.
- Fig. 5: Reported uPA capture level after injection of each sample was plotted as a function of the aptamer concentration relative to the response of 20 nM uPA alone for uPAapt-21, uPAapt-26, uPAapt-08 and the sequence-unrelated control oligonucleotide Con (number of records N=2).

### Cited literature

Botkjaer, Kenneth A.; Deryugina, Elena I.; Dupont, Daniel M.; Gårdsvoll, Henrik; Bekes, Erin M.; Thuesen, Cathrine K. et al. (2012): Targeting tumor cell invasion and dissemination in vivo by an aptamer that inhibits urokinase-type plasminogen activator through a novel multifunctional mechanism. In: Molecular cancer research: MCR 10 (12), S. 1532-1543. DOI: 10.1158/1541-7786.MCR-12-0349.
Dupont, Daniel Miotto; Madsen, Jeppe Buur; Kristensen, Thomas; Bodker, Julie Stove; Blouse, Grant Ellsworth; Wind, Troels; Andreasen, Peter Andre (2009): Biochemical properties of plasminogen activator inhibitor-1. In: Frontiers in bioscience (Landmark edition) 14, S. 1337-1361. DOI: 10.2741/3312.
Dupont, Daniel Miotto; Madsen, Jeppe Buur; Hartmann, Roland Karl; Tavitian, Bertrand; Ducongé, Frédéric; Kjems, Jørgen; Andreasen, Peter Andre (2010): Serum-stable RNA aptamers to urokinase-type plasminogen activator blocking receptor binding. In: RNA (New York, N. Y.) 16 (12), S. 2360-2369. DOI: 10.1261/rna.2338210.
Higazi, A.; Cohen, R. L.; Henkin, J.; Kniss, D.; Schwartz, B. S.; Cines, D. B. (1995): Enhancement of the enzymatic activity of single-chain urokinase plasminogen activator by soluble urokinase receptor. In: The Journal of biological chemistry 270 (29), S. 17375-17380. DOI: 10.1074/jbc.270.29.17375.
Ma, D.; Gerard, R. D.; Li, X. Y.; Alizadeh, H.; Niederkorn, J. Y. (1997): Inhibition of metastasis of intraocular melanomas by adenovirus-mediated gene transfer of plasminogen activator inhibitor type 1 (PAI-1) in an athymic mouse model. In: Blood 90 (7), S. 2738-2746. Mahmood, Niaz; Mihalcioiu, Catalin; Rabbani, Shafaat A. (2018): Multifaceted Role of the Urokinase-Type Plasminogen Activator (uPA) and Its Receptor (uPAR): Diagnostic, Prognostic, and Therapeutic Applications. In: Frontiers in oncology 8, S. 24. DOI: 10.3389/fonc.2018.00024.
Mekkawy, Ahmed H.; Pourgholami, Mohammad H.; Morris, David L. (2014): Involvement of urokinase-type plasminogen activator system in cancer: an overview. In: Medicinal research reviews 34 (5), S. 918-956. DOI: 10.1002/med.21308.
Muehlenweg, B.; Sperl, S.; Magdolen, V.; Schmitt, M.; Harbeck, N. (2001): Interference with the urokinase plasminogen activator system: a promising therapy concept for solid tumours. In: Expert opinion on biological therapy 1 (4), S. 683-691. DOI: 10.1517/14712598.1.4.683. Nobuhara, M.; Sakamaki, M.; Ohnishi, H.; Suzuki, Y. (1981): A comparative study of high molecular weight urokinase and low molecular weight urokinase. In: Journal of biochemistry 90 (1), S. 225-232. DOI: 10.1093/oxfordjournals.jbchem.a133453.
Ossowski, L.; Russo-Payne, H.; Wilson, E. L. (1991): Inhibition of urokinase-type plasminogen activator by antibodies: the effect on dissemination of a human tumor in the nude mouse. In: Cancer research 51 (1), S. 274-281.
Petersen, L. C.; Lund, L. R.; Nielsen, L. S.; Danø, K.; Skriver, L. (1988): One-chain urokinase-type plasminogen activator from human sarcoma cells is a proenzyme with little or no intrinsic activity. In: The Journal of biological chemistry 263 (23), S. 11189-11195.
Sato, Sumito; Kopitz, Charlotte; Schmalix, Wolfgang A.; Muehlenweg, Bernd; Kessler, Horst; Schmitt, Manfred et al. (2002): High-affinity urokinase-derived cyclic peptides inhibiting urokinase/urokinase receptor-interaction: effects on tumor growth and spread. In: FEBSletters 528 (1-3), S. 212-216. DOI: 10.1016/s0014-5793(02)03311-2.
Schmitt, Manfred; Harbeck, Nadia; Brünner, Nils; Jänicke, Fritz; Meisner, Christoph; Mühlenweg, Bernd et al. (2011): Cancer therapy trials employing level-of-evidence-1 disease forecast cancer biomarkers uPA and its inhibitor PAI-1. In: Expert review of molecular diagnostics 11 (6), S. 617-634. DOI: 10.1586/erm.11.47.
Skrypina, Natalia A.; Savochkina, Larissa P.; Beabealashvilli, Robert Sh (2004): In vitro selection of single-stranded DNA aptamers that bind human pro-urokinase. In: Nucleosides, nucleotides & nucleic acids 23 (6-7), S. 891-893. DOI: 10.1081/NCN-200026037

## Claims

1. DNA aptamer comprising a polynucleotide sequence having at least 70 % identity to a sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11 for use as a medicament.

2. The DNA aptamer for use according to claim 1 in treatment of cancer and/or prevention of cancer in a subject.

3. The DNA aptamer for use according to claim 1 or 2, which is able to inhibit binding of Urokinase type plasminogen activator (uPA) with Urokinase type plasminogen activator receptor (uPAR) and optionally able to inhibit activity of Urokinase type plasminogen activator (uPA).

4. The DNA aptamer for use according to any one of claims 1 to 3 comprising a polynucleotide sequence having at least 70 % identity to a sequence selected from the group consisting of SEQ ID NO: 1, SEQ ID NO:3, SEQ ID NO:4, SEQ ID NO:5 and SEQ ID NO:6.

5. The DNA aptamer for use according to any one of claims 1 to 4, wherein the sequence identity is least 80 %, preferably at least 90 %, more preferably at least 95 %, more preferably at least 97 %.

6. The DNA aptamer for use according to any one of claims 2 to 5, wherein the cancer is bladder cancer or breast cancer.

7. The DNA aptamer for use according to any one of claims 1 to 6, wherein the subject is a human.

8. A DNA aptamer comprising a polynucleotide sequence having at least 70 % identity to a sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11.

9. The DNA aptamer according to claim 8, wherein the sequence identity is least 80 %, preferably at least 90 %, more preferably at least 95 %, more preferably at least 97 %.

10. A method for identifying a subject benefiting from cancer treatment with a DNA aptamer comprising:
(a) contacting a sample comprising cancer cells of said subject with a DNA aptamer comprising a polynucleotide sequence having at least 70 % identity to a sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11;
(b) determining the degree of inhibition of binding of Urokinase type plasminogen activator (uPA) with Urokinase type plasminogen activator receptor (uPAR) and/or the degree of inhibition of Urokinase type plasminogen activator (uPA) in the cancer cells of step (a),
(c) comparing the degree of inhibition determined in step (b) to a reference, preferably from control treated cancer cells of said subject, and
(d) based on the result of step (c), identifying a subject benefiting from treatment with a polynucleotide having at least 70 % identity to a sequence selected from the group consisting of SEQ ID NO: 1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11.

11. The method according to claim 10, which is an *ex vivo* or *in vitro* method, preferably an *in vitro* method.

12. A kit comprising (i) a DNA aptamer comprising a polynucleotide having at least 70 % identity to a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10 and SEQ ID NO:11 and (ii) a means for determining inhibition of binding of Urokinase type plasminogen activator (uPA) with Urokinase type plasminogen activator receptor (uPAR) and/or a means for determining an inhibition of Urokinase type plasminogen activator (uPA) in a sample.
